# EUROPEAN PATENT SPECIFICATION

(11) **EP 3 052 202 B1**
(45) Date of publication and mention of the grant of the patent: **06.03.2019**
(21) Application number: 14780505.5
(22) Date of filing: 03.10.2014
(51) Int. Cl.: A61Q 19/04, A61K 8/97

(54) **SKIN TANNING EXTRACT**
HAUTBRÄUNUNGSEXTRAKT
EXTRAIT DE BRONZAGE DE LA PEAU

(30) Priority: 04.10.2013 EP 13187302
(43) Date of publication of application: 10.08.2016
(73) Proprietor: DSM IP Assets B.V., 6411 TE Heerlen (NL)
(72) Inventor: CAMPICHE, Remo, CH-4303 Kaiseraugst (CH); IMFELD, Dominik, CH-4303 Kaiseraugst (CH); VOEGELI, Rainer, CH-4303 Kaiseraugst (CH)
(74) Representative: Berg, Katja
(86) International application number: PCT/EP2014/071223
(87) International publication number: WO 2015/049366

(56) References cited:
- WO-A2-99/66881
- WO-A2-2012/116990
- DATABASE GNPD [Online] MINTEL; February 2012 (2012-02), WALA Heilmittel, Dr. Hauschka: "Quince Body Moisturizer", XP002721562, Database accession no. 1739318
- DATABASE GNPD [Online] MINTEL; September 2010 (2010-09), Weleda: "Soothing Face Oil", XP002721563, Database accession no. 1387126

## Description

The present invention relates to the use of a *Prunus spinosa* blossom extract as a skin tanning agent. Furthermore, the present invention relates to the cosmetic topical application of a *Prunus spinosa* blossom extract formulated into a composition comprising a cosmetically acceptable carrier for skin tanning. Finally, the invention relates to the use of a *Prunus spinosa* blossom extract for enhancement of melanin formation in human skin cells.

It is increasingly important for humans to look healthy, and a tanned skin is always a sign of good health. One method of obtaining a skin tan is to expose skin to UV radiation causing direct DNA damage to the skin, which the body naturally combats and seeks to repair. In the process of repairing the damage and protect the skin, the body creates and releases the brown-colored pigment called melanin into the skin's cells, which gives the skin a darker tone. Melanin is produced by cells called melanocytes and protects the body from direct and indirect DNA damage by absorbing an excess of solar radiation. However, as exposure to UV radiation may have detrimental health effects such as sunburn or even skin cancer. Thus, many people prefer to use chemical products which can produce a tanning result without exposure to ultraviolet radiation.

Today, most of the cosmetic products intended for artificially tanning of the skin comprise carbonyl compounds such as dihydroxyacetone (DHA) or erythrulose which cause a chemical reaction with the amino acids in the dead layer on the skin surface thus forming colored species. However, the drawback of these compounds is that the tan produced therewith, in contrast to sun tanned skin, does not protect against UV-radiation and the achieved skin color does not correspond exactly to naturally sun tanned skin and the tan often is uneven.

Thus, there is an ongoing need for compounds which stimulate the production of melanin in skin melanocytes similarly to the production of melanin stimulated by DNA damage induced by UVB-radiation, which in turn leads to the development of a natural skin tan.

WO99/66881 describes compositions containing plant extracts, which have a high radical protection factor. It is however silent with regard to blossom extracts.

WO2012116990 discloses the use of Steviol as well as derivatives thereof as skin tanning agent, however is also silent with regard to blossom extracts.

Cosmetic products comprising a Prunus spinosa blossom extract are already known and used to purify and soften the skin or to care delicate and sensitive skin such as the Soothing Face Oil from Weleda, or the 'Lait Corporel au Coing' from Dr. Hauschka.

Surprisingly it has been found that the use of a *Prunus spinosa* blossom extract overcomes the drawbacks of the prior art and activates the melanin formation in human skin melanocytes and can thus be used for sunless tanning. The formulations containing a *Prunus spinosa* blossom extract also have the advantage of being slightly colored on application to the skin and thus of being able to be dosed such as to visualize the zone of application of the product. Furthermore, the change in pigmentation (i.e. increased melanin content) will render the skin better-prepared to deal with direct sunlight, thereby reducing the risk and/or severity of skin problems such as sunburns, premature wrinkling and aging, etc.

Thus, in one embodiment, the invention relates to the use of a *Prunus spinosa* blossom extract according to claim 1 as skin tanning agent, and/or as an agent for the enhancement of the natural skin tan.

Due to the increased skin tan (skin pigmentation) following the application of a *Prunus spinosa* blossom extract, the skin is better protected against skin damages due to ultraviolet radiation. Thus, another aspect of this invention relates to the use of a *Prunus spinosa* blossom extract according to claim 3 for preventing or reducing the risk of sun burns, for enhancing the photoprotection to bot UV A and UV B induced skin damage, for increasing the skin's UV protection, for delaying the onset or severity of photoageing and/ or for preventing or reducing immune suppression.

In a further embodiment the present invention relates to the use of a *Prunus spinosa* blossom extract according to claim 2 for the enhancement of melanin formation in human epidermal melanocytes, in particular when applied topically.

*Prunus spinosa* (blackthorn or sloe) is a species of *Prunus* native to Europe, western Asia, and locally in Northwest Africa. *Prunus spinosa* is a large deciduous shrub or small tree growing to 5 meters tall, with blackish bark and dense, stiff, spiny branches. The leaves are oval, 2 - 4.5 centimeters long and 1.2 - 2 centimeters broad, with a serrated margin. The flowers are 1.5 centimeters diameter, with five creamy-white petals; they are produced shortly before the leaves in early spring, and are hermaphroditic and insect-pollinated.

The *Prunus spinosa* blossom extract may be used in free or in encapsulated form, for example in lipid vesicles such as liposomes such as e.g. disclosed in WO97/25970, in nanosomes or in cyclodextrins.

The *Prunus spinosa* blossom extract used for the present invention can be sourced from chemical suppliers such as Dr. Hans-Jörg Treiber, Waibstadt, Germany, or Shaanxi Joryherb Bio Technology Co. Ltd. Xi'an, China. Moreover, it can be prepared by extraction according to methods well known to a person skilled in the art as documented in Olszewska and Wolbis (2001), Acta Pol. Pharm 58(5).367-372.

The extract according to the present invention can be a standard water extract, or an extract prepared with water and up to 50 vol.% ethanol. More preferably, in view of its superior melanonegenesis stimulation effect on human melanocytes, it is a 50 vol.% water/ethanol extract. Moreover, the extract is characterized by the presence of kaempferol, quercetin, and glycosylated derivatives comprising: kaempferol 3-O-alpha-L-arabinofuranoside, kaempferol 3-O-alpha-L-ramnopyranoside, kaempferol 7-O-alpha-L-ramnopyranoside, kaempferol 3-O-beta-D-xylopyranoside, kaempferol 3-O-(2"-E-p-coumaroyl)-alpha-L-arabinofuranoside, kaempferol 3,7-di-O-alpha-L-rhamnopyranoside, quercetin 3-O-alpha-L-arabinofuranoside, quercetin 3-O-alpha-L-arabinopyranoside, quercetin 3-O-alpha-L-rhamnopyranoside, quercetin 3-O-beta-D-xylo-pyranoside, quercetin 3-O-beta-glucopyranoside, kaempferol and quercetin 3-O-(4"-beta-D-glucopyranosyl)-alpha-L-rhamnopyranosides.

The use according to the present invention is especially attractive, since many humans have a special interest in cosmetic treatments considered as "natural" with mild effects and without major side effects.

*Prunus spinosa* blossom extract is preferably used in the form of a topical composition. The topical compositions advantageously comprise from 0.0001 to 20 wt.-% of a *Prunus spinosa* blossom extract, (II) based on the total weight of the composition. Advantageously, the topical compositions comprise from 0.005 to 5 wt.-%, such as particularly from 0.05 to 2 wt.-%, of a *Prunus spinosa* blossom extract based on the total weight of the composition.

Thus, in another embodiment the invention also relates to a method of increasing the skin tan said method comprising topically applying onto the skin of an individual seeking such treatment an effective amount of a cosmetic composition comprising a *Prunus spinosa* blossom extract in an amount ranging from 0.0001-20 wt.-% based on the total weight of the composition formulated into a cosmetically acceptable carrier. Preferably, the *Prunus spinosa* blossom extract is used in an amount ranging from 0.005-5 wt.-% and even more preferably in an amount ranging from 0.05-2 wt.-%.

Due to the increased skin tan (skin pigmentation) the skin of humans which topically apply a *Prunus spinosa* blossom extract is better protected against skin damages due to ultraviolet radiation. Thus, the invention furthermore relates to a method of
Preventing or reducing the risk of sun burns
Increasing the skin's UV protection
Delaying the onset or severity of photoageing (i.e. as anti-ageing agent),
Preventing or reducing immune suppression and/ or
Enhancing the photoprotection to both UV A and UV B induced skin damage. said method comprising topically applying onto the skin of an individual seeking such treatment an effective amount of a cosmetic composition comprising a *Prunus spinosa* blossom extract in an amount ranging from 0.0001-20 wt.-% based on the total weight of the composition formulated into a cosmetically acceptable carrier. Preferably, the *Prunus spinosa* blossom extract is used in an amount ranging from 0.005-5 wt.-% and even more preferably in an amount ranging from 0.05-2 wt.-%. Said method is particularly interesting for fairly tanned individuals or immunosuppressed patients.

The term "effective amount" means an amount necessary to obtain a desired physiological effect.

The physiological effect may be achieved by one single dose or by repeated doses. The dosage administered may, of course, vary depending upon known factors, such as the physiological characteristics of the particular composition; the age, health and weight of the recipient; the nature and extent of the symptoms; the kind of concurrent treatment; the frequency of treatment; and/or the effect desired and can be adjusted by a person skilled in the art. Preferably, 1 g cream per cm² skin is applied once a day.

The term "topical composition" as used herein denotes to any composition suitable for the topical application to mammalian keratinous tissue such as in particular to human skin. In particular, the topical compositions according to the present invention are cosmetic compositions that can be topically applied to mammalian keratinous tissue, particularly to human skin. Thus, the topical compositions according to the present invention are in particular cosmetic skin care compositions comprising at least one compound of formula (I) such as in particular of formula (II) and a cosmetically acceptable carrier.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Preparations", Verlag für chemische Industrie (ed. H. Ziolkowsky), 4th edition, 1992.

The term cosmetically acceptable carrier refers to all carriers and/or excipients and/ or diluents conventionally used in cosmetic compositions.

It is advantageous if the topical compositions according to the invention exhibit a pH of 8 or less such as in particular a pH in the range of 2.5 to 8, more in particular in the range of 3 to 7.5. The pH of the topical composition can be adjusted with conventional acids, bases or buffering solutions according to methods well known to a person skilled in the art.

According to a further advantageous embodiment, the topical compositions according to the present invention contain in addition at least one stabilizer and/or at least one photoprotective agent and/or at least one wetting agent and/or at least one penetrant and/or at least one additional coloring agent.

In order to improve the stability of the *Prunus spinosa* blossom extract, the compositions according to the invention advantageously include one or more stabilizers such as e.g. antioxidants or chelating agents.

Suitable antioxidants/ chelating agents to be incorporated into the topical compositions according to the present invention are basically all known antioxidants/ chelating agents usually formulated into topical and in particular cosmetic compositions. Especially preferred are antioxidants/ chelating agents chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol to µmol/ kg), additionally (metal)-chelators (such as α-hydroxyfatty acids (citric acid, lactic acid, malic acid), palmic-, phytinic acid, lactoferrin), β-hydroxyacids, huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, Tetradibutyl Pentaerithrityl Hydroxyhydrocinnamate (Tinogard TT), Tetrabutyl Ethylidinebisphenol (Tinogard NOA), Octadecyl Di-t-butyl-4-hydroxyhydrocinnamate (Tinogard TS) trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients, or enzymes such as superoxide dismutase, catalase or similar, or activators of such enzymes. The one or more antioxidant/ chelating agent may be present in an amount of at least 0.01 wt.- % such as in an amount of 0.01 to about 10 wt.- % and particularly in an amount of 0.1 to about 1 wt.-% based on the total weight of the composition.

Particularly suited antioxidants for the use in the topical compositions according to the invention encompass vitamin E and its derivatives such as particularly tocopheryl acetate. Tocopheryl acetate may be present in the topical compositions in an amount from about 0.05 wt.-% to 25 wt.-%, in particular 0.05 wt.-% to 5 wt.-%. Another vitamin E derivative of interest is tocopheryl linoleate. Tocopheryl linoleate may be present in the topical composition in an amount from about 0.05 wt.-% to 25 wt.-% in particular 0.05 wt.-% to 5 wt.-%.

Another suitable antioxidant is vitamin A and/or its derivatives. In particular retinoid derivatives such as retinyl palmitate or retinyl propionate is used in the topical compositions according to the invention in an amount of 0.01 to 5 wt.-%, in particular 0.01 to 0.3 wt.-%. The vitamin A and/ or its derivatives can also be used in an encapsulated form.

Another particular suitable antioxidant is Vitamin C (ascorbic acid) and/or its derivatives. In particular ascorbyl phosphate such as Stay C (sodium ascorbyl monophosphate) Mg ascorbylphosphate and/ or Ascorbylglucoside is used in the topical compositions according to the invention in an amount of 0.1 to 5 wt.-% in particular of 0.1 to 2 wt.-%.

Suitable photoprotective agents which may be incorporated into the topical compositons according to the present invention include conventional UV-filter substances. The UV-filter substances are advantageously selected from among acrylates such as 2-ethylhexyl 2-cyano-3,3-diphenylacrylate (octocrylene, PARSOL® 340), ethyl 2-cyano-3,3-diphenylacrylate and the like; camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as polysilicone-15 (PARSOL® SLX); drometrizole trisiloxane (Mexoryl® XL); imidazole derivatives such as e.g. 2-phenyl benzimidazole sulfonic acid and its salts (PARSOL® HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL® EHS, Neo Heliopan® OS), isooctyl salicylate or homomenthyl salicylate (homosalate, PARSOL® HMS, Neo Heliopan® HMS) and the like; triazine derivatives such as ethylhexyl triazone (Uvinul® T-150), diethylhexyl butamido triazone (Uvasorb® HEB), 2,4,6-Tris-(biphenyl)1,3,5-triazine and the like, merocyanines as e.g. disclosed in DE10 2007 024 345 on page 4, paragraph 19 which are incorporated by reference herein, encapsulated UV-filters such as encapsulated ethylhexyl methoxycinnamate (Eusolex® UV-pearls) or microcapsules loaded with UV-filters as e.g. disclosed in EP 1471995 and the like; dibenzoylmethane derivatives such as 4-tert.-butyl-4'-methoxydibenzoyl-methane (PARSOL® 1789) or isopropyldibenzoylmethane and the like; benzotriazole derivatives such as 2,2'-methylene-bis-(6-(2H-benzotriazole-2-yl)-4-(1,1,3,3,-tetramethylbutyl)-phenol (Tinosorb® M) and the like; bis-ethylhexyloxyphenol methoxyphenyl triazine (Tinosorb® S) and the like; phenylene-1,4-bis-benzimidazolsulfonic acids or salts such as 2,2-(1,4-phenylene)bis-(1H-benzimidazol-4,6-disulfonic acid) (Neo Heliopan® AP); amino substituted hydroxybenzophenones such as 2-(4-Diethylamino-2-hydroxy-benzoyl)-benzoic acid hexylester (Uvinul® A plus) or 1,1'-(1,4-piperazinediyl)bis[1-[2-[4-(diethylamino)-2-hydroxybenzoyl]phenyl]-methanone (CAS No 919803-06-8); Ionic UV-A filters as described in the International Patent Publication WO2005080341 A1; pigments such as microparticulated ZnO or TiO₂ and the like. The term "microparticulated" refers to a particle size from about 5 nm to about 200 nm, particularly from about 15 nm to about 100 nm. The pigments may also be coated by other metal oxides such as e.g. aluminum or zirconium oxides or by organic coatings such as e.g. polyols, methicone, aluminum stearate, alkyl silane. Such coatings are well known in the art. Furthermore, the pigments (ZnO, TiO₂) can be used in the form of commercially available oily or aqueous pre-dispersions. These pre-dispersions may further contain a dispersing aid and/ or solubilisator.

Particularly preferred UV-filter substances are the commercially available and widely used UV-filter substances octocrylene (PARSOL® 340), 4-methyl benzylidene camphor (PARSOL® 5000), ethylhexyl methoxycinnamate (PARSOL® MCX), ethylhexyl triazone (Uvinul® T-150), diethylhexyl butamido triazone (Uvasorb® HEB), 2,2'-methylene-bis-(6-(2H-benzotriazole-2-yl)-4-(1,1,3,3,-tetramethylbutyl)-phenol (Tinosorb® M), bis-ethylhexyl-oxyphenol methoxyphenyl triazine (Tinosorb® S), 2,2-(1,4-phenylene)bis-(1H-benzimidazol-4,6-disulfonic acid (NeoHeliopan® AP), 2-(4-Diethylamino-2-hydroxybenzoyl)-benzoic acid hexylester (Uvinul® A plus), 1,1'-(1,4-piperazinediyl)bis[1-[2-[4-(diethylamino)-2-hydroxybenzoyl]phenyl]-methanone (CAS No 919803-06-8), polysilicone-15 (PARSOL® SLX), 2-phenyl benzimidazole sulfonic acid (PARSOL® HS), ethylhexyl salicylate (PARSOL® EHS), homomenthyl salicylate (PARSOL® HMS), Benzophenone-3 (Uvinul® M 40), Benzophenone-4 (Uvinul® MS 40), Butyl Methoxydibenzoyl Methane (Parsol® 1789), Terephtalidene dicampher Sulfonic Acid (Mexoryl® SX), Drometrizole Trisiloxane (Mexoryl® XL), microfine zinc or titanium dioxide such as in particular PARSOL® TX as well as mixtures thereof.

The photoprotective agents (in total) are generally present in the topical compositions according to the invention comprising at least one compound of formula (I) such as in particular of formula (II) in proportions ranging from 0.1 to 30 wt.-%, preferably ranging from 0.2 to 15 wt.-%, most preferably ranging from 0.5 to 10 wt.-% based on the total weight of the composition.

In order to increase the remanence of the skin color and/or the homogeneity of the color, the compositions according to the invention comprising a *Prunus spinosa* blossom extract may additionally comprise at least a wetting agent and/or penetrant such as for instance urea, hydroxyethylurea, polyols such as glycerol, alkylene glycols such as propylene glycol or butylene glycol, or alkylene glycol alkyl ethers such as propylene glycol monomethyl ether.

In order to adjust the color obtained via the tanning process according to the invention and to better adapt it to the various types of skin tone, the topical compositions comprising a *Prunus spinosa* blossom extract in accordance with the present invention may also comprise one or more additional coloring agents.

The additional coloring agents may be selected especially from natural and synthetic direct dyes. They may be organic or mineral dyes. The mineral dyes may be, for example, iron oxide pigments whose mean elementary particle size is less than 100 nm, such as those described in EP-966,953. The natural or synthetic liposoluble organic dyes are, for example, DC Red 17, DC Red 21, DC Red 27, DC Green 6, DC Yellow 11, DC Violet 2, DC Orange 5, Sudan red, carotenes ([beta]-carotene or lycopene), xanthophylls (capsanthin, capsorubin or lutein), palm oil, Sudan brown, quinoline yellow, annatto and curcumin.

The natural or synthetic water-soluble dyes are, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper-containing chlorophylline, methylene blue, anthocyanins (enocyanin, black carrot, hibiscus or elder) and riboflavin.

The dyes may also be selected from among anthraquinones, caramel, carmine, carbon black, azulene blues, methoxalene, trioxalene, guajazulene, chamuzulene, rose Bengal, cosine 10B, cyanosin, daphinine, juglone, lawsone, extracts of fermented soya, of algae, of fungi or of microorganisms, flavylium salts not substituted in position 3, for instance those described in EP-1-172,091, extracts of Gesneria fulgens, Blechum procerum or Saxifraga and pigments that may be obtained by extraction with an organic or aqueous-organic solvent of a culture medium of micromycetes of the Monascus type.

These dyes may also be selected from among indole derivatives, for instance the monohydroxyindoles as described in FR-2,651,126 (i.e.: 4-, 5-, 6- or 7-hydroxyindole) or the dihydroxyindoles as described in EP-B-0-425,324 (i.e.: 5,6-dihydroxyindole, 2-methyl-5,6-dihydroxyindole, 3-methyl-5,6-dihydroxyindole or 2,3-dimethyl-5,6-dihydroxyindole).

Further coloring agents are e.g. self-tanning agents such as dihydroxyacetone (DHA), erythrulose and/ or melanin derivates which may be incorporated into the topical compositions according to the invention. Advantageously the topical compositions according to the present invention additionally contain dihydroxyacetone (DHA) and/ or erythrulose in an amount of 1 to 10 wt.-% based on the total weight of the composition according to the invention.

The topical compositions according to the invention may further comprise biological actives selected from general activators of melanogenesis like tyrosinase activators, peptide hormones, MCR-1 agonists, MITF stimulators, cAMP stimulators (forskulin), cAMP-activators (caffeine) and neurotrophins.

Preferred tyrosinase activators are any substance which increases tyrosinase expression or enzyme activity, like e.g. glycyrrhizin from the root of licorice or glycyrrhetic acid.

The topical compositions according to the invention preferably contain at least one tyrosinase activator in an amount (in total) of 0.01 to 1 wt.-%, preferably 0.1 to 0.5 wt.-% based on the total weight of the composition.

Peptide hormones belonging to the group of melanocortins are the preferred peptide hormones including ACTH, alpha-MSH, beta-MSH and gamma-MSH or their synthetically modified analogues Melanotan I and II; these peptides are all cleavage products of a large precursor peptide called pro-opiomelanocortin (POMC). Alpha-MSH is the most important melanocortin for pigmentation. The melanocyte-stimulating hormones (collectively referred to as MSH or intermedins) are a class of peptide hormones that in nature are produced by cells in the intermediate lobe of the pituitary gland. They stimulate the production and release of melanin (melanogenesis) by melanocytes in skin. Therefore, they will be advantageously combined with the *Prunus spinosa* blossom extract.

Of particular interest is the combination a *Prunus spinosa* blossom extract according to this invention with tri or tetra-peptides representing the recognition sequence in the synthetically modified analogues Melanotan I and II, which is His-D-Phe-Arg-Trp. These tri- or the tetra-peptides may have a hydrophobic modification on the C- or N-terminus for better skin penetration.

A list of further peptides, hydrophobically modified or not for combination with the *Prunus spinosa* blossom extract of this invention can be found in US2008200396.

The topical compositions according to the invention comprising a *Prunus spinosa* blossom extract may also comprise additional active agents selected especially from moisturizers, further skin tanning agents, desquamating agents, agents for improving the barrier function, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, tensioning agents, lipo-restructuring agents, slimming agents, agents for promoting the cutaneous capillary circulation, calmatives and/or anti-irritants, sebo-regulators or anti-seborrhoeic agents, astringents, cicatrizing agents, anti-inflammatory agents and anti-acne agents.

One skilled in the art will select the said active agent(s) as a function of the effect desired on the skin.

Particularly suitable moisturizers for the incorporation into the topical compositions according to the invention are glycerine, lactic acid and/ or lactates, in particular sodium lactate, butylene glycol, propylene glycol, biosaccaride gum-1, glycine soja, ethylhexyloxyglycerin, pyrrolidoncarboxy acid, hydroxyethylurea and urea. It is further advantageous to use polymeric moisturizer such as water soluble or water gelifiable polysaccharides. In particular advantageous are e.g. hyaluronic acid, chitosan, Pentavitin and/ or a polysaccharid rich in fucose [CAS No 178463-23-5, commercially available as Fucogel®1000 by SOLABIA S.A.]. The moisturizers can also be used as anti-ageing ingredients such as e.g. for the treatment of photo-aged skin.

The topical compositions according to the invention preferably contain at least one moisturizer in an amount (in total) of 0.1 to 20 wt.-%, preferably 0.5 to 10 wt.-% based on the total weight of the composition.

Further examples of cosmetically active ingredients suitable to be used in the topical composition according to the invention comprising a *Prunus spinosa* blossom extract comprise peptides (e.g., Matrixyl™ [pentapeptide derivative]), oligopeptides, wax-based synthetic peptides (e.g., octyl palmitate and tribehenin and sorbitan isostearate and palmitoyl-oligopeptide), glycerol, alpha-glycosylrutin, natural or synthetic flavanoids or isoflavanoids, creatine, creatinine, guanidine (e.g. amino guanidine); vitamins and derivatives thereof such as vitamin C (ascorbic acid), vitamin A (e.g., retinoid derivatives such as retinyl palmitate or retinyl propionate), vitamin E (e.g., tocopherol acetate), vitamin B₃ (e.g. niacinamide) and vitamin B₅ (e.g. panthenol), vitamin B₆ and vitamin B₁₂, biotin, folic acid; anti-acne actives or medicaments (e.g. resorcinol, salicylic acid, and the like); antioxidants (e.g. phytosterols, lipoic acid); flavonoids (e.g. isoflavones, phytoestrogens); skin soothing and healing agents such as aloe vera extract, allantoin and the like; agents suitable for aesthetic purposes such as essential oils, fragrances, skin sensates, opacifiers, aromatic compounds (e.g., clove oil, menthol, camphor, eucalyptus oil, and eugenol), desquamatory actives, hydroxy acids such as AHA acids, poly unsaturated fatty acids, radical scavengers, farnesol, antifungal actives in particular bisabolol, alkyldiols such as 1,2-pentanediol, hexanediol or 1,2-octanediol, phytol, polyols such as phytanetriol, ceramides and pseudoceramides, amino acids, protein hydrolysates, polyunsaturated fatty acids, plant extracts like kinetin, DNA or RNA and their fragmentation products, carbohydrates, conjugated fatty acids, carnitin, carnosine, biochinonen, phytofluen, phytoen, and their corresponding derivatives and co-enzyme Q10 (ubiquinone) without being limited thereto.

The additional cosmetically active ingredient is typically included in an amount of at least 0.001 wt.-% based on the total weight of the topical composition. Generally, an amount of about 0.001 wt.-% to about 30 wt.-%, preferably from about 0.001 wt.-% to about 10 wt.-% of an additional cosmetically active agent is used.

Particularly preferred examples of ingredients to be used in the compositions according to the invention are vitamin C (ascorbic acid) and/or its derivatives (e.g. ascorbyl phosphate such as Stay C (sodium ascorbyl monophosphate) from DSM Nutritional Products Ltd.), vitamin A and/or its derivatives (e.g., retinoid derivatives such as retinyl palmitate or retinyl propionate), vitamin E and/or its derivatives (e.g., tocopherol acetate), vitamin B₆, vitamin B₁₂, biotin and/ or co-enzyme Q10.

The topical cosmetic compositions of the invention can also contain usual cosmetic or pharmaceutical adjuvants and additives, such as preservatives, film forming agents, fatty substances/ oils and/ or waxes, water, organic solvents, silicones, thickeners, softeners, emulsifiers, antifoaming agents, aesthetic components such as fragrances, surfactants, fillers, sequestering agents, anionic, cationic, nonionic or amphoteric polymers or mixtures thereof, propellants, acidifying or basifying agents, complexing agents, colorings/colorants, abrasives, absorbents, essential oils, skin sensates, astringents, perfumes or any other ingredients usually formulated into cosmetic compositions such as alcohols, polyols or electrolytes. Such cosmetic ingredients commonly used in the skin care industry, which are suitable for use in the compositions of the present invention, are e.g. described in the CTFA Cosmetic Ingredient Handbook, Second Edition (1992) without being limited thereto.

The necessary amounts of the cosmetic and pharmaceutical adjuvants and additives can - based on the desired product form- easily be chosen by a skilled person in this field and will be illustrated in the examples, without being limited hereto.

The usual cosmetic adjuvants and additives such as e.g. emulsifiers, thickeners, surface active ingredients and film formers can show synergistic effects which can be determined by the expert in the field with normal trials, or with the usual considerations regarding the formulation of cosmetic composition.

The fatty substances can be an oil or a wax, or mixtures thereof. By the term "oil" is intended a compound which is liquid at ambient temperature. By the term "wax" is intended a compound which is solid or substantially solid at ambient temperature and for which the melting point is generally greater than 35° C.

Exemplary oils are mineral oils (liquid paraffin); vegetable oils (sweet almond, macadamia, blackcurrant seed or jojoba oil); synthetic oils, such as perhydrosqualene, fatty alcohols, acids or esters (such as the C₁₂₋₁₅ alkyl benzoate marketed under the trademark "Finsolv TN" by Finetex, octyl palmitate, isopropyl lanolate or triglycerides, including those of capric/caprylic acids), or oxyethylenated or oxypropylenated fatty esters and ethers; silicone oils (cyclomethicone, polydimethylsiloxanes or PDMS); fluorinated oils; polyalkylenes and their mixtures.

Preferably the oils used in the compositions according to the invention are selected from the list of polar oils such as the lecitines and fatty acid triglycerides, namely triglycerinester of saturated or unsaturated, branched or linear alkanoic acids with a chain length of 8 to 24, particularly 12 to 18C-atoms. The fatty acid triglycerides may preferably be selected from the group of synthetic, semi synthetic and natural oils such as e.g. cocoglyceride, olive oil, sunflower oil, soy bean oil, peanut oil, palm oil, sweet almond oil macadamia oil, coconut oil etc.

Further particularly suitable are natural waxes such as bees wax, shea butter, and/ or lanolin. Further particularly suitable polar oils according to the present invention may be selected from the group of esters of saturated or unsaturated, branched or linear alkanoic acids with a chain length of 3 to 30 C-atoms and saturated or unsaturated, branched or linear alcohols with a chain length of 3 to 30C-atoms as well as from the group of esters from aromatic carbonic acids and saturated or unsaturated, branched or linear alcohols with a chain length of 3 to 30C-atoms. Such ester oils are particularly selected from the group of phenylethylbenzoate, octylpalmitate, octylcocoate, octylisostearate, octyldodeceylmyristate, octyldodecanol, cetearylisononanoate, isopropylmyristate, isopropylpalmitate, isopropylstearate, isopropyloleate, n-butylstearate, n-hexyllaurate, n-decyloleate, isooctylstearate, isononylstearate, isononylisononanoate, 2-ethylhexylpalmitate, 2-ethylhexyllaurate, 2-hexyldecylstearate, 2-octyldodecylpalmitate, stearylheptanoate, isopropyl lauroyl sarkosinate, oleyloleate, oleylerucate, erucyloleate, erucylerucate, tridecylstearate, tridecyltrimellitate as well as synthetic and semi synthetic and natural mixtures of such esters such as e.g. jojoba oil.

Further particularly suitable oils may be selected from the group of dialkyl ether and dialkylcarbonates such as particularly dicaprylylether (Cetiol OE) and/ or dicaprylylcarbonate, (e.g. available as Cetiol CC at Cognis).

Further particularly suitable oils may be selected from the group of isoeikosan, neopentylglykoldiheptanoate, propylenglykoldicaprylaet caprylate/dicaprate, caprylic/ capric/ diglyceryl succinate, butylene glyckol dicaprylate/dicaprate, C₁₂₋₁₃-Alkyllactate, Di-C₁₂₋₁₃-alkyltartrate, triisostearin, dipentaerythrityl hexacaprylate hexacaprate, propylenglykolmonoisostearate, tricaprylin and dimethylisosorbid.

It is particularly advantageous if the oil phase of the topical compositions according to the invention contains an amount of C₁₂₋₁₅-alkylbenzoate or consists essentially thereof.

Further particularly suitable oily components are e.g. butyloctylsalicylate (e.g. Hallbrite BHB from CP Hall), hexadecylbenzoate and butyloctylbenzoate as well as mixtures therof (e.g. Hallstar AB).

The topical compositions according to the present invention may also contain apolar oils such as e.g. branched or linear hydrocarbons and waxes, in particular mineral oil, vaseline (Petrolatum), paraffin oil, squalan and squalen, polyolefins, hydrogenated polyisobutenes, C₁₃₋₁₆ isoparaffin and isohexadecan. Within the group of polyolefins polydecenes are preferred.

Exemplary waxy compounds in particular suitable for the use in the compositions according to the invention are paraffin wax, carnauba wax, beeswax or hydrogenated castor oil.

Exemplary organic solvents in particular suitable for the use in the compositions according to the invention include the lower alcohols and polyols having at most 8 carbon atoms. In particular the compositions according to the invention comprise ethanol in an amount of 5 to 40 wt.-% based on the total weight of the composition.

The thickeners are advantageously selected, in particular, from among the cross linked polyacrylic acids or modified or unmodified guar gums and celluloses, such as hydroxypropylated guar gum, methylhydroxy-ethylcellulose and hydroxypropylmethylcellulose.

Suitable film forming agents include polymers on the basis of PVP such as in particular copolymers of polyvinylpyrrolidon e.g. PVP hexadecen copolymer and PVP eicosen copolymer which are available as Antaron V216 and Antaron V220 at GAF Chemicals corporations. Further suitable film forming agents include polymeric film formers such as sodiumpolystyrenesulfonate (e.g. Flexan 130 from National Starch and Chemical Corp.) and/ or polyisobuten (e.g. Rewopal PIB1000 from Rewo). Further suitable polymers are e.g. polyacrylamide (Seppigel 305), polyvinylalkohole, PVP, PVP/VA copolymers, polyglycols and acrylate/octylacralymid copolymers (e.g. Dermacryl 79). Further suitable is the use of hydrated castor oil dimerdilinoleat (CAS 646054-62-8, INCI Hydrogenated Castor Oil Dimer Dilinoleate), or PPG-3 Benzylethermyristate (CAS 403517-45-3).

The topical compositions according to the invention may further comprise one or several compounds from the group of siloxanes elastomers listed in order to enhance the water resistance and/ or enhance the light protection factor such as in particular siloxanes elastomers in the form of spherical powders with the INCI nomenclature Dimethicone/Vinyl Dimethicone Crosspolymer, such as e.g. DOW CORNING 9506 Powder (by Dow corning).

It is particularly advantageous if the siloxane elastomer is used in combination with hydrocarbon oils, synthetic oils, synthetic esters, synthetic ether or mixtures thereof.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

Preferred topical compositions according to the invention are skin care preparation such as particularly skin-tanning preparations (i.e. compositions for the artificial/sunless tanning and/or browning of human skin).

Examples of skin care preparations are, in particular body oils, body lotions, body gels, treatment creams, skin protection ointments, moisturizing gels or moisturizing sprays,

The topical compositions are applied at least several times per week, preferably at least once per day, and more preferably applied at least twice a day such as e.g. once in the morning and once in the evening. Applications should be for a chronic period of time, i.e. at least one week, preferably for at least two weeks, and more preferably for at least 4 weeks in order to observe results.

The topical compositions according to the present invention can also be used to achieve a cosmetic effect, which could be beautifying skin, in particular the treatment or prophylaxis of wrinkles or dry skin or sensitive skin or any symptoms caused by negative developments of the physiological homeostasis of healthy skin, moisturizing the skin, thickening of the epidermis, treatment or prophylaxis of acne, inhibition of senescence of skin cells, prevention or treatment of photodamage, prevention or treatment of oxidative stress phenomena, prevention or treatment of cellulite, prevention or treatment of pigmentation disorders and/or to even and/or darken the skin tone, prevention and treatment of disturbances in ceramide and lipid synthesis, prevention of excess sebum production, reduction of activities of matrix metallo proteases or other proteases in the skin, treatment and prevention of inflammatory skin conditions including atopic eczema, polymorphic light eruption, psoriasis, vertiligo, prevention and treatment of itchy or irritated skin, strengthening and/or protection of nails or a skin cleansing effect.

The invention is further illustrated by the Examples which follow without being limited thereto.

### EXAMPLES

**Example 1:** Induction of melanin synthesis by *Prunus spinosa* blossom extract in human epidermal melanocytes and mouse melanoma cell line B16F10.

### Quantification of melanin synthesis in cell culture:

Mouse melanoma cells (B16F10) from internal working cell bank, passage numbers 4-6 or normal neonatal human epidermal melanocytes NHM (HEMn-MP) from moderately-pigmented tissue, passage numbers 4-6, were seeded in 96-well cell culture plates (20'000 cells per well) and grown to sub-confluence for three days in DMEM culture medium (for B16F10 cells), supplemented with 5% FCS, or in a 1:2 mixture of M2-Medium (Clonetics) and Promocell-Medium together 'Culture medium' (for HEMn-MP cells). Culture medium was exchanged with Culture Medium containing the Prunus spinosa extract (50% ethanol/water extract) and 24hrs later, culture medium containing extract was replaced with freshly prepared one. Then melanogenesis was progressed for another three days in an incubator at a temperature of 37°C and at 10% CO₂ atmosphere (for B16F10 cells) and at 5% CO₂ atmosphere (for HEMn-MP cells). Total melanin was extracted using 1.7M KOH for 1hour at room temperature. We measured melanin content at 405 nm in an absorbance plate reader.

### Results

Melanin synthesis by blackthorn extract in mouse melanoma cell line B16F10. Untreated control is 100% melanin synthesis. Each concentration was tested 4 times:

| Extract | Concentration (dry extract) | % melanin synthesis | STDEV (%) |
|---|---|---|---|
| Blackthorn blossom extract | 0.005% | 155 | +/-1 |
| | 0.005% | 160 | +/-12 |
| | 0.005% | 221 | +/-28 |
| | 0.005% | 241 | +/-5 |
| | 0.0005% | 126 | +/-7 |
| | 0.0005% | 135 | +/-17 |
| | 0.0005% | 167 | +/-21 |
| | 0.0005% | 181 | +/-11 |
| | 0.00005% | 106 | +/-15 |
| | 0.00005% | 131 | +/-9 |
| | 0.00005% | 164 | +/-3 |
| | 0.00005% | 133 | +/-17 |

Melanin synthesis of blackthorn extract in human epidermal melanocytes. Untreated control is 100% melanin synthesis:

| Extract | Concentration (dry extract) | % melanin synthesis | STDEV (%) |
|---|---|---|---|
| Blackthorn blossom extract | 0.005% | 129 | +/-17 |
| | 0.005% | 144 | +/-4 |
| | 0.0005% | 111 | +/-33 |
| | 0.0005% | 108 | +/-3 |

**Example 2:** Skin tanning preparations comprising *Prunus spinosa* blossom extract

| **O/W-Emulsions** | **2.1** | **2.2** |
|---|---|---|
| | **Wt.-%** | **Wt.-%** |
| Cetearyl Alcohol + Sodium Cetylstearylsulfat | 3.00 | 2.00 |
| Glycerylstearat SE | 2.00 | 4.00 |
| Octyldodecanol | 2.00 | 2.00 |
| C12-15 Alkylbenzoate | 1.00 | 1.00 |
| C13-16 Isoparaffin | 3.00 | 3.00 |
| Caprylic acid -/Capric acid triglyceride | 2.00 | 2.00 |
| Glycerine | 5.00 | 6.00 |
| Dimethicone | 0.50 | 0.50 |
| Sodium ascorbylphosphate | 0.10 | - |
| Ethylhexylsalicylate | 0.50 | 0.50 |
| Glycyrrhetic acid | - | 0.10 |
| *Prunus spinosa* blossom extract | 1.00 | 1.50 |
| Grape seed oil | 0.50 | 0.50 |
| Dihydroxyacetone | - | 2.00 |
| Paraffinum Liquidum + Ginkgo Biloba Extract | 0.25 | 0.25 |
| Citric acid | 0.09 | 0.09 |
| Sodium citrate | 0.17 | 0.17 |
| Xanthan gum | - | 0.10 |
| Ammonium Acryloyldimethyltaurate/VP Copolymer | 0.40 | - |
| Carbomer | - | 0.30 |
| Parabene | 0.30 | 0.30 |
| Phenoxyethanol | 0.50 | 0.50 |
| Alcohol Denat. | 3.50 | 3.50 |
| Perfume | q.s | q.s |
| Water | ad 100 | ad 100 |

| **Sprayable emulsions** | **2.3** | **2.4** |
|---|---|---|
| | **Wt.-%** | **Wt.-%** |
| Isoceteth-20 | 5.00 | 3.00 |
| Glycerylisostearate | 3.00 | 2.00 |
| Mineral Oil | 5.00 | 4.00 |
| Glycerine | 4.00 | 5.00 |
| Tocopherylacetate | 0.50 | 0.40 |
| *Prunus spinosa* blossom extract | 0.20 | 0.50 |
| Natriumcitrate | 0.40 | 0.40 |
| Phenoxyethanol | 0.40 | 0.40 |
| Citric acid | 0.20 | 0.20 |
| DMDM Hydantoin | 0.20 | 0.20 |
| Perfume | q.s. | q.s. |
| Water | ad 100 | ad 100 |

The pH of the formulation is adjusted to about pH 7

| **O/W-Emulsions** | **2.5** | **2.6** | **2.7** |
|---|---|---|---|
| | **wt.-%** | **wt.-%** | **wt.-%** |
| Stearic acid | 2.00 | 3.00 | 3.00 |
| Glycerylstearat | 2.00 | 2.00 | 1.00 |
| Sorbitanstearat | | 1.00 | - |
| Dicaprylyl Ether | 3.00 | 3.00 | - |
| Caprylic acid -/Capric acid triglyceride | 3.00 | 3.00 | - |
| Cetearyl Alcohol | 2.00 | 2.00 | - |
| Cetyl Alcohol | - | - | 1.00 |
| Stearyl Alcohol | - | - | 3.00 |
| Hydrogenated Coco-Glycerides | - | - | 4.00 |
| Mineral Oil | - | - | 3.00 |
| PEG-100 stearat | - | 1.00 | 0.50 |
| Trisodium EDTA | - | - | 1.00 |
| Glycerine | 4.00 | 6.00 | 10.00 |
| Dimethicone | - | - | 1.00 |
| Glycyrrhetic acid | 0.20 | - | - |
| *Prunus spinosa* blossom extract | 0.20 | 0.50 | 0.10 |
| Erythrulose | - | 4.00 | |
| Phenoxyethanol | 0.40 | 0.40 | 0.40 |
| Parabene | 0.20 | 0.20 | 0.20 |
| Citric acid | - | - | 0.09 |
| Carbomer | 0.20 | 0.20 | 0.20 |
| Perfume | q.s. | q.s. | q.s. |
| Water | ad 100 | ad 100 | ad 100 |

| **O/W-Emulsions** | **2.8** | **2.9** |
|---|---|---|
| | **Wt.-%** | **wt.-%** |
| Glycerylstearate | 3.00 | 4.00 |
| C12-15 Alkylbenzoate | 4.00 | 4.00 |
| Caprylic acid -/Capric acid triglyceride | 3.00 | 2.50 |
| Isopropylstearat | 2.00 | 2.50 |
| Cetyl Alcohol | 2.00 | 2.00 |
| Stearyl Alcohol | 2.00 | 2.00 |
| Glycerin | 3.00 | 5.00 |
| Dimethicone | 0.50 | 2.00 |
| Glycyrrhetic acid | - | 0.10 |
| *Prunus spinosa* blossom extract | 1.00 | 0.50 |
| Phenoxyethanol | 0.40 | 0.40 |
| Parabene | 0.20 | 0.20 |
| Carbomer | 0.10 | 0.10 |
| Perfume | q.s. | q.s. |
| Water | ad 100 | ad 100 |

The pH of the formulation is adjusted to about pH 7

| **Tanning Spray** | **2.10** |
|---|---|
| | **Wt.-%** |
| Butyl methoxydibenzoylmethane | 5.00 |
| Octocrylene | 10.00 |
| Homosalate | 5.00 |
| Glycerine | 0.50 |
| *Prunus spinosa* blossom extract | 0.50 |
| Perfume | q.s. |
| Ethanol | ad 100 |

The pH of the formulation is adjusted to about pH 7

| **Emulsions-Fluid** | **2.11** |
|---|---|
| | **Wt.-%** |
| Stearic acid | 2.00 |
| Dicaprylylether | 3.00 |
| Octyldodecanol | 2.00 |
| C12-15 Alkylbenzoate | 4.00 |
| Cetylalcohol | 2.00 |
| Cetearyl Ethylhexanoate + Isopropylmyristate | 2.00 |
| Glycerine | 5.00 |
| Ethylhexylmethoxycinnamate | 2.00 |
| TiO2 | 1.00 |
| Cetylpalmitate | 1.00 |
| Glyceryl Stearate | 1.00 |
| Phenoxyethanol | 0.40 |
| Butyl Methoxydibenzoxylmethane | 2.00 |
| Perfume | q.s. |
| EDTA | 0.20 |
| Carbomer | 0.20 |
| Magnesium Aluminium Silicate | 0.20 |
| Paraben | 0.20 |
| Vitamin E Acetat | 0.10 |
| *Prunus spinosa* blossom extract | 0.10 |
| Paraben | 0.05 |
| BHT | 0.05 |
| DHA | 1.00 |

| **O/W-Emulsion: night cream** | **2.12** |
|---|---|
| | **Wt.-%** |
| Glycerylstearatcitrate | 2.00 |
| Stearylalcohol | 2.00 |
| Cetylalcohol | 2.00 |
| Hydrogenated Coco Glyceride | 1.00 |
| Caprylic acid -/Capric acid triglyceride | 3.00 |
| Ethylhexylkcoco fatty acid esters | 2.00 |
| Dicaprylylether | 2.00 |
| C12-15 Alkylbenzoate | 3.00 |
| Tocopherylacetate | 1.00 |
| Ubichinon (Coenzyme Q10) | 0.10 |
| Sodium ascorbylphosphate | 0.10 |
| *Prunus spinosa* blossom extract | 1.00 |
| Parabene | 0.40 |
| Methylpropandiol | 1.00 |
| Carrageenan | 0.10 |
| Carbomer | 0.20 |
| Tapioca starch | 2.00 |
| EDTA | 0.20 |
| Glycerine | 5.00 |
| Waster and/ or oil soluble dyes | 0.05 |
| Filling agents/Additives | 0.50 |
| Perfume | q.s. |
| Water | ad 100 |

The pH of the formulation is adjusted to about pH 6.5

| **O/W-Emulsion: day cream** | **2.13** | **2.14** |
|---|---|---|
| | **Wt.-%** | **Wt.-%** |
| PEG-40-Stearat | 1.00 | 1.00 |
| Glycerylstearate | 3.00 | 3.00 |
| Cetearylalcohol | 2.00 | 2.00 |
| Dimethicone | 1.00 | 1.00 |
| Hydrogenated Coco Glvcerides | 2.00 | 2.00 |
| Caprylic acid -/Capric acid triglyceride | 2.00 | 2.00 |
| Octyldodecanole | 2.00 | 2.00 |
| Dicaprylylcarbonate | 2.00 | 2.00 |
| C12-15 Alkylbenzoate | 3.00 | 3.00 |
| Ethylhexyl methoxycinnamate | 4.00 | 4.00 |
| Butyl methoxydibenzoylmethane | 2.00 | 2.00 |
| Tocopherylacetate | 1.00 | 1.00 |
| Panthenol | 0.50 | 0.50 |
| Sodium ascorbylphosphate | 0.10 | 0.10 |
| Ammonium Acryloyldimethyltaurate/VP Copolymer | - | 0.40 |
| Glycyrrhetic acid | - | 0.10 |
| *Prunus spinosa* blossom extract | 1.00 | 0.50 |
| Nylon-12 | 3.00 | - |
| Distarch phosphate | - | 2.00 |
| Parabene | 0.40 | 0.40 |
| Methylpropandiol | 1.00 | 1.00 |
| Carbomer | 0.20 | 0.20 |
| Xanthan gum | 0.10 | 0.10 |
| EDTA | 0.20 | 0.20 |
| Glycerine | 8.00 | 8.00 |
| Tapioca starch | 0.05 | 0.05 |
| Filling agents/Additives | 0.30 | 0.30 |
| Perfume | q.s. | q.s. |
| Water | ad 100 | ad 100 |

The pH of the formulation is adjusted to about pH 6.5

| **O/W-Emulsion: facial cream** | **2.15** | **2.16** |
|---|---|---|
| | **Wt.-%** | **wt.-%** |
| Polyglyceryl-3-Methylglucosedistearate | 2.00 | 2.00 |
| Sorbitanstearate | 3.00 | 3.00 |
| Cetylalcohol | 2.00 | 2.00 |
| Myristylmyristate | 1.00 | 1.00 |
| Dicaprylylether | 3.00 | 3.00 |
| Octyldodecanol | 2.00 | 2.00 |
| C12-15 Alkylbenzoate | 3.00 | 3.00 |
| Cetearyl Ethylhexanoat + Isopropylmyristate | 2.00 | 2.00 |
| Ethylhexyl methoxycinnamate | 2.00 | 2.00 |
| Ethylhexyltriazone | 1.00 | 1.00 |
| Butyl Methoxydibenzoxylmethane | 2.00 | 2.00 |
| Magnesium Aluminium Silicate | 0.20 | 0.20 |
| Glycerine | 5.00 | 5.00 |
| Phenoxyethanole | 0.40 | 0.40 |
| Parabene | 0.30 | 0.30 |
| Vitamin E Acetate | 0.10 | 0.10 |
| Glycyrrhetic acid | - | 0.10 |
| *Prunus spinosa* blossom extract | 1.50 | 1.00 |
| BHT | 0.05 | 0.05 |
| EDTA | 0.20 | 0.20 |
| Carbomer | 2.00 | 0.20 |
| Perfume | q.s. | q.s. |
| Water | ad 100 | ad 100 |

The pH of the formulation is adjusted to about pH 7

## Claims

1. A *Prunus spinosa* blossom extract for use as skin tanning agent, and/or as an agent for the enhancement of the natural skin tan, wherein the *Prunus spinosa* blossom extract is prepared by extraction with water and up to 50% ethanol.

2. A *Prunus spinosa* blossom extract for use in a topical method for the enhancement of melanin formation in human epidermal melanocytes, wherein the *Prunus spinosa* blossom extract is prepared by extraction with water and up to 50% ethanol.

3. A *Prunus spinosa* blossom extract for use in preventing or reducing the risk of sun burns, for use in enhancing the photoprotection to both UV A and UV B induced skin damage, for use in increasing the skin's UV protection, for use in delaying the onset or severity of photoageing and/ or for use in preventing or reducing immune suppression, wherein the *Prunus spinosa* blossom extract is prepared by extraction with water and up to 50% ethanol.

4. The Prunus spinose blossom extract for the use according to anyone of claims 1 to 3, wherein the extract is a 50 vol.% water/ethanol extract.

5. The *Prunus spinosa* blossom extract for the use according to any of claims 1 to 4, wherein, the *Prunus spinosa* blossom extract comprises quercetin, kaempfol and glycosylated derivatives.

6. The *Prunus spinosa* blossom extract for the use according to claim 5, wherein, the glycosylated derivatives are mixtures of quercetin and kaempferol 3-O-alpha-L-arabinofuranoside, 3-O-alpha-L-ramnopyranoside, 3-O-beta-D-xylopyranoside, 3-0-(4"-beta-D-glucopyranosyl)-alpha-L-rhamnopyranosides, kaempferol 7-O-alpha-L-ramnopyranoside, kaempferol 3-O-(2"-E-p-coumaroyl)-alpha-L-arabinofuranoside, kaempferol 3,7-di-O-alpha-L-rhamnopyranoside, quercetin 3-O-alpha-L-arabinopyranoside, and quercetin 3-O-beta-glucopyranoside,

7. The *Prunus spinosa* blossom extract for the use according to any of claims 1 to 6 in topical compositions, which comprise from 0.0001 - 20 wt.-% of *Prunus spinosa* blossom extract based on the total weight of the composition.

8. The *Prunus spinosa* blossom extract for the use according to claim 7, wherein the amount of *Prunus spinosa* blossom extract in the topical composition is selected in the range of 0.05-2 wt.-% based on the total weight of the composition.

9. The *Prunus spinosa* blossom extract for the use according to any of the claims 1 to 8, wherein the topical composition has a pH of 8 or less.

10. The *Prunus spinosa* blossom extract for the use according to claim 9, wherein the pH of the topical composition is selected in the range from 3 to 7.5.

11. The *Prunus spinosa* blossom extract for the use according to any one of claims 1 to 10 wherein the topical composition further comprises at least one stabilizer and/or at least one photoprotective agent and/or at least one wetting agent and/or at least one penetrant and/or at least one coloring agent.

12. The *Prunus spinosa* blossom extract for the use according to claim 11 wherein the coloring agent is dihydroxyacetone and/ or erythrulose.

13. The *Prunus spinosa* blossom extract for the use according to claim 11, wherein the at least one photoprotective agent is selected from the group consisting of octocrylene, 4-methyl benzylidene, ethylhexyl methoxycinnamate, ethylhexyl triazone, diethylhexyl butamido triazone, 2,2'-methylene-bis-(6-(2H-benzotriazole-2-yl)-4-(1,1,3,3,-tetramethylbutyl)-phenol), bis-ethylhexyl-oxyphenol methoxyphenyl triazine, 2,2-(1,4-phenylene)bis-(1H-benzimidazol-4,6-disulfonic acid, 2-(4-Diethylamino-2-hydroxy-benzoyl)-benzoic acid hexylester, 1,1'-(1,4-piperazinediyl)bis[1-[2-[4-(diethylamino)-2-hydroxybenzoyl]- phenyl]-methanone, polysilicone-15, 2-phenyl benzimidazole sulfonic acid, ethylhexyl salicylate, homomenthyl Salicylate, Benzophenone-3, Benzophenone-4, Butyl Methoxydibenzoyl Methane, Terephtalidene dicampher Sulfonic Acid, Drometrizole Trisiloxane and microfine zinc or titanium dioxide as well as mixtures thereof.

## Patentansprüche

1. *Prunus-spinosa*-Blütenextrakt zur Verwendung als Hautbräunungsmittel und/oder als Mittel zur Verstärkung der natürlichen Hautbräune, wobei der *Prunus-spinosa-*Blütenextrakt durch Extraktion mit Wasser und bis zu 50% Ethanol hergestellt wird.

2. *Prunus-spinosa*-Blütenextrakt zur Verwendung in einem topischen Verfahren zur Verstärkung der Melaninbildung in humanen epidermalen Melanozyten, wobei der *Prunus-spinosa*-Blütenextrakt durch Extraktion mit Wasser und bis zu 50% Ethanol hergestellt wird.

3. *Prunus-spinosa*-Blütenextrakt zur Verwendung bei der Prävention oder dem Senken des Risikos von Sonnenbrand, zur Verwendung bei der Verstärkung des Lichtschutzes gegenüber sowohl durch UV A als auch UV B induzierte Hautschäden, zur Verwendung bei der Erhöhung des UV-Schutzes der Haut, zur Verwendung bei der Verzögerung des Einsetzens oder dem Schweregrad von Lichtalterung und/oder zur Verwendung bei der Prävention oder der Verminderung von Immunsuppression, wobei der *Prunus-spinosa*-Blütenextrakt durch Extraktion mit Wasser und bis zu 50% Ethanol hergestellt wird.

4. *Prunus-spinosa*-Blütenextrakt zur Verwendung nach einem der Ansprüche 1 bis 3, wobei es sich bei dem Extrakt um einen 50 vol.-%igen Wasser/Ethanol-Extrakt handelt.

5. *Prunus-spinosa*-Blütenextrakt zur Verwendung nach einem der Ansprüche 1 bis 4, wobei der *Prunus-spinosa-*Blütenextrakt Quercetin, Kämpferol und glykosylierte Derivate umfasst.

6. *Prunus-spinosa*-Blütenextrakt zur Verwendung nach Anspruch 5, wobei es sich bei den glykosylierten Derivaten um Mischungen von Quercetin und Kämpferol-3-O-alpha-L-arabinofuranosid, 3-O-alpha-L-Rhamnopyranosid, 3-O-beta-D-Xylopyranosid, 3-O-(4"-beta-D-Glucopyranosil)-alpha-L-rhamnopyranosiden, Kämpferol-7-O-alpha-L-rhamnopyranosid, Kämpferol-3-O-(2"-E-p-coumaroyl)-alpha-L-arabinofuranosid, Kämpferol-3,7-di-O-alpha-L-rhamnopyranosid, Quercetin-3-O-alpha-L-arabinopyranosid und Quercetin-3-O-beta-glucopyranosid handelt.

7. *Prunus-spinosa*-Blütenextrakt zur Verwendung nach einem der Ansprüche 1 bis 6 in topischen Zusammensetzungen, welche 0,0001 - 20 Gew.-% *Prunus-spinosa*-Blütenextrakt, bezogen auf das Gesamtgewicht der Zusammensetzung, umfassen.

8. *Prunus-spinosa*-Blütenextrakt zur Verwendung nach Anspruch 7, wobei die Menge an *Prunus-spinosa-*Blütenextrakt in der topischen Zusammensetzung aus dem Bereich von 0,05-2 Gew.-%, bezogen auf das Gesamtgewicht der Zusammensetzung, gewählt ist.

9. *Prunus-spinosa*-Blütenextrakt zur Verwendung nach einem der Ansprüche 1 bis 8, wobei die topische Zusammensetzung einen pH-Wert von 8 oder weniger aufweist.

10. *Prunus-spinosa*-Blütenextrakt zur Verwendung nach Anspruch 9, wobei der pH-Wert der topischen Zusammensetzung aus dem Bereich von 3 bis 7,5 gewählt ist.

11. *Prunus-spinosa*-Blütenextrakt zur Verwendung nach einem der Ansprüche 1 bis 10, wobei die topische Zusammensetzung weiterhin mindestens einen Stabilisator und/oder mindestens ein Lichtschutzmittel und/oder mindestens ein Netzmittel und/oder mindestens ein Penetrationsmittel und/oder mindestens ein Färbemittel umfasst.

12. *Prunus-spinosa*-Blütenextrakt zur Verwendung nach Anspruch 11, wobei es sich bei dem Färbemittel um Dihydroxyaceton und/oder Erythrulose handelt.

13. *Prunus-spinosa*-Blütenextrakt zur Verwendung nach Anspruch 11, wobei das mindestens eine Lichtschutzmittel aus der aus Octocrylen, 4-Methylbenzyliden, Methoxyzimtsäureethylhexylester, Ethylhexyltriazon, Diethylhexylbutamidotriazon, 2,2'-Methylenbis-(6-(2H-benzotriazol-2-yl)-4-(1,1,3,3-tetramethylbutyl)phenol), Bisethylhexyloxyphenolmethoxyphenyltriazin, 2,2-(1,4-Phenylen)bis-(1H-benzimidazol)-4,6-disulfonsäure, 2-(4-Diethylamino-2-hydroxybenzoyl)benzoesäurehexylester, 1,1'-(1,4-Piperazindiyl)bis[1-[2-[4-(diethylamino)-2-hydroxybenzoyl]phenyl]methanon, Polysilicon-15, 2-Phenylbenzimidazolsulfonsäure, Ethylhexylsalicylat, Homomenthylsalicylat, Benzophenon-3, Benzophenon-4, Butylmethoxydibenzoylmethan, Terephthalidendicamphersulfonsäure, Drometrizoltrisiloxan und mikrofeinem Zink oder Titandioxid sowie Mischungen davon bestehenden Gruppe ausgewählt ist.

## Revendications

1. Extrait de fleurs de *Prunus spinosa* pour une utilisation comme agent bronzant pour la peau, et/ou comme agent pour l'amélioration du bronzage naturel de la peau, où l'extrait de fleurs de *Prunus spinosa* est préparé par une extraction par de l'eau et jusqu'à 50% d'éthanol.

2. Extrait de fleurs de *Prunus spinosa* pour une utilisation dans une méthode topique d'amélioration de la formation de mélanine dans les mélanocytes épidermiques humains, où l'extrait de fleurs de *Prunus spinosa* est préparé par une extraction par de l'eau et jusqu'à 50% d'éthanol.

3. Extrait de fleurs de *Prunus spinosa* pour une utilisation dans la prévention ou la réduction du risque d'érythème solaire, pour une utilisation dans la photoprotection contre des lésions de la peau induites par les UVA ainsi que les UVB, pour une utilisation dans l'augmentation de la protection de la peau contre les UV, pour une utilisation dans le retardement du déclenchement ou de la gravité d'un photo-vieillissement et/ou pour une utilisation dans la prévention ou la réduction d'une immunosuppression, où l'extrait de fleurs de *Prunus spinosa* est préparé par une extraction par de l'eau et jusqu'à 50% d'éthanol.

4. Extrait de fleurs de *Prunus spinosa* pour une utilisation selon l'une quelconque des revendications 1 à 3, où l'extrait est un extrait eau/éthanol à 50% vol.

5. Extrait de fleurs de *Prunus spinosa* pour une utilisation selon l'une quelconque des revendications 1 à 4, où l'extrait de fleurs de *Prunus spinosa* comprend de la quercétine, du kaempférol et des dérivés glycosylés.

6. Extrait de fleurs de *Prunus spinosa* pour une utilisation selon la revendication 5, où les dérivés glycosylés sont des mélanges de quercétine et de kaempférol 3-O-alpha-L-arabinofuranoside, 3-O-alpha-L-ramnopyranoside, 3-O-bêta-D-xylopyranoside, 3-O-(4"-bêta-D-glucopyranosyl)-alpha-L-rhamnopyranosides, kaempférol 7-O-alpha-L-ramnopyranoside, kaempférol 3-O-(2"-E-p-coumaroyl)-alpha-L-arabinofuranoside, kaempférol 3,7-di-O-alpha-L-rhamnopyranoside, quercétine 3-O-alpha-L-arabinopyranoside, et quercétine 3-O-bêta-glucopyranoside.

7. Extrait de fleurs de *Prunus spinosa* pour une utilisation selon l'une quelconque des revendications 1 à 6 dans des compositions topiques, comprenant 0,0001-20% en poids d'extrait de fleurs de *Prunus spinosa,* sur la base du poids total de la composition.

8. Extrait de fleurs de *Prunus spinosa* pour une utilisation selon la revendication 7, où la quantité d'extrait de fleurs de *Prunus spinosa* dans la composition topique est choisie dans la plage de 0,05-2% en poids, sur la base du poids total de la composition.

9. Extrait de fleurs de *Prunus spinosa* pour une utilisation selon l'une quelconque des revendications 1 à 8, où la composition topique possède un pH de 8 ou moins.

10. Extrait de fleurs de *Prunus spinosa* pour une utilisation selon la revendication 9, où le pH de la composition topique est choisi dans la plage allant de 3 à 7,5.

11. Extrait de fleurs de *Prunus spinosa* pour une utilisation selon l'une quelconque des revendications 1 à 10, où la composition topique comprend en outre au moins un stabilisant et/ou au moins un agent photo-protecteur et/ou au moins un agent mouillant et/ou au moins un agent de pénétration et/ou au moins un agent colorant.

12. Extrait de fleurs de *Prunus spinosa* pour une utilisation selon la revendication 11, où l'agent colorant est la dihydroxyacétone et/ou l'érythrulose.

13. Extrait de fleurs de *Prunus spinosa* pour une utilisation selon la revendication 11, où le au moins un agent photo-protecteur est choisi dans le groupe constitué par l'octocrylène, le 4-méthylbenzylidène, le méthoxycinnamate d'éthylhexyle, l'éthylhexyltriazone, la diéthylhexylbutamidotriazone, le 2,2'-méthylène-bis-(6-(2H-benzotriazole-2-yl)-4-(1,1,3,3-tétraméthylbutyl)-phénol), la bis-éthylhexyloxyphénolméthoxyphényl-triazine, l'acide 2,2-(1,4-phénylène)bis-(1H-benzimidazol-4,6-disulfonique, l'acide 2-(4-diéthyl-amino-2-hydroxybenzoyl)benzoïque hexylester, la 1,1'-(1,4-pipérazinediyl)bis[1-[2-[4-(diéthylamino)-2-hydroxybenzoyl]phényl]méthanone, la polysilicone-15, l'acide 2-phénylbenzimidazolesulfonique, le salicylate d'éthylhexyle, le salicylate d'homomenthyle, la benzophénone-3, la benzophénone-4, le butylméthoxy-dibenzoylméthane, l'acide téréphtalidène dicamphre-sulfonique, le drométrizole trisiloxane et le zinc microfin ou le dioxyde de titane, ainsi que des mélanges de ceux-ci.
